(19) Europäisches Patentamt
European Patent Office
Office européen des brevets

(11) EP 0 804 147 B1

(12) # FASCICULE DE BREVET EUROPEEN

(45) Date de publication et mention de la délivrance du brevet:
**07.10.1998 Bulletin 1998/41**

(21) Numéro de dépôt: **95922574.9**

(22) Date de dépôt: **07.06.1995**

(51) Int. Cl.$^6$: **A61K 7/48**, A61K 31/155, A61K 31/19

(86) Numéro de dépôt international:
**PCT/FR95/00740**

(87) Numéro de publication internationale:
**WO 95/33443 (14.12.1995 Gazette 1995/53)**

(54) **COMPOSITION DERMATO-COSMETOLOGIQUE**
DERMATO-KOSMETOLOGISCHES PRÄPARAT
DERMATO-COSMETOLOGIC COMPOSITION

(84) Etats contractants désignés:
**AT BE CH DE DK ES FR GB GR IE IT LI LU MC NL PT SE**

(30) Priorité: **08.06.1994 FR 9406994**

(43) Date de publication de la demande:
**05.11.1997 Bulletin 1997/45**

(73) Titulaire:
**PIERRE FABRE DERMO-COSMETIQUE
92100 Boulogne (FR)**

(72) Inventeurs:
- **COUVAL, Emmanuelle
31520 Ramonville-Saint-Agne (FR)**
- **FERAL, Marie-Hélène
31320 Castanet-Tolosan (FR)**
- **GOURNAY, Anne
31400 Toulouse (FR)**
- **LAGARDE, Isabelle
31520 Ramonville-Saint-Agne (FR)**
- **PEYROT, Nicole
31400 Toulouse (FR)**

(74) Mandataire: **Ahner, Francis et al
CABINET REGIMBEAU
26, avenue Kléber
75116 Paris (FR)**

(56) Documents cités:
**DE-A- 2 740 349** **DE-A- 3 941 534**
**US-A- 4 163 800**

- **PATENT ABSTRACTS OF JAPAN vol. 12, no. 128 (C-489) & JP,A,62 249 908 (LION CORP.)**

EP 0 804 147 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne de nouvelles compositions utiles en dermatologie et/ou cosmétologie et leurs applications au traitement et/ou à l'amélioration d'un ensemble de troubles cutanés.

Les acides $\alpha$ ou $\beta$ hydroxylés ont été utilisés en applications topiques à titre de principe actif ou pour améliorer l'activité d'autres composés.

La demande EP- 273 202 a décrit l'emploi d'acides hydroxy carboxyliques, considérés comme inactifs par eux-mêmes, pour augmenter l'activité de compositions dermatologiques sur les rides.

Le brevet FR- 2 363 326 a proposé l'utilisation du produit de réaction d'acide hydroxycarboxylique avec une base organique ou minérale pour le traitement des peaux sèches.

La présente invention a pour objet une composition dermatocosmétologique, caractérisée en ce qu'elle contient à titre de principe actif au moins un sel obtenu par réaction de la guanidine ou d'un de ses sels et d'un acide $\alpha$ ou $\beta$ hydroxylé choisi dans le groupe comprenant : l'acide citrique, l'acide glycolique, l'acide gluconique, l'acide glucuronique, l'acide galacturonique, l'acide $\alpha$ hydroxybutyrique, l'acide $\alpha$ hydroxyisobutyrique, l'acide lactique, l'acide malique, l'acide mandélique, l'acide galactarique, l'acide $\beta$ phényl lactique, l'acide glucarique, l'acide tartrique, l'acide tartronique, l'acide $\beta$ hydroxy butyrique, l'acide salicylique.

En effet, la Demanderesse a montré que l'utilisation d'un sel de guanidine, permet d'obtenir des compositions présentant des propriétés améliorées par rapport aux acides ou aux autres sels généralement décrits.

Ceci pourrait être dû à la structure particulière de la guanidine, qui est une base forte, de formule $HN = C (NH_2)_2$. Elle peut se présenter plus particulièrement sous forme de carbonate :

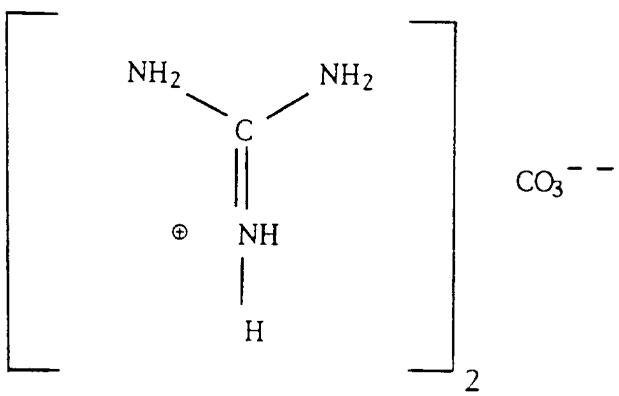

L'acide hydroxylé peut être présent à une concentration comprise entre environ 5 et 70% en poids par rapport au poids total de la composition.

Selon un aspect préféré, le sel d'acide hydroxylique présent dans les compositions selon l'invention est le glycolate de guanidine. On obtient alors des compositions efficaces avec des concentrations en glycolate de guanidine comprises entre environ 0,5% et 15% en poids par rapport au poids total de la composition.

Le glycolate de guanidine en applications topiques, présente des propriétés d'hydratation de la peau, aussi bien in vitro qu'in vivo, qui se sont montrées très supérieures a celles des autres acides hydroxylés ou de leurs sels ; on a ainsi trouvé que le remplacement du lactate d'ammonium, qui est un produit de référence dans ce domaine, par du glycolate de guanidine, permet de diminuer de plus de la moitié les concentrations en principes actifs pour obtenir un effet analogue.

Selon un autre aspect, le glycolate de guanidine a démontré d'excellentes propriétés kératorégulatrices, supérieures à celles du lactate d'ammonium qui est couramment utilisé comme kératolytique.

Ces propriétés seront avantageusement mises à profit lors de l'utilisation du glycolate de guanidine ou des compositions le contenant, pour la préparation d'un médicament destiné au traitement d'une affection choisie dans le groupe comprenant : acné, ichtyose, kératoses séborrhéiques, kératoses actiniques, lentigos séniles, verrues et hyperkératinisations.

Le glycolate de guanidine peut, selon un des aspects de l'invention, remplir une fonction de conservateur dans les compositions le contenant. Cette fonction peut être assurée seule, ou en même temps que les fonctions d'hydratation et/ou de kératorégulation. Ceci est particulièrement avantageux car on peut ainsi réduire ou éviter l'ajout d'autres conservateurs, toujours susceptibles d'engendrer des problèmes de tolérance sur des peaux dont l'intégrité est déjà atteinte; les concentrations et le type de conservateurs utilisables sont limités notamment par la CEE. Le glycolate de guanidine peut également jouer ce rôle dans des compositions contenant d'autres principes actifs.

Ces compositions peuvent être à usage cosmétique ou pharmaceutique, sous forme de crèmes, émulsions, lotions, shampooings, gels nettoyants ou poudre. La Demanderesse a montré qu'une crème contenant par exemple 13% de glycolate de guanidine s'auto-protège contre les contaminations microbiennes et rend inutile la présence d'autres conservateurs. Des résultats satisfaisants sont également obtenus pour des formules contenant :

| | |
|---|---|
| . solution à 13% de glycolate de guanidine | 7 à 10% |
| . EDTA | 0,2% |

En outre, le glycolate de guanidine est actif sur des bactéries impliquées dans la formation d'odeurs corporelles en particulier au niveau des aisselles, telles que les Corynébactéries. Cette activité bactéricide permet l'utilisation du glycolate de guanidine comme déodorant corporel, dans des compositions qui empêchent notamment la prolifération de Corynebacterium aquaticum, tout en respectant la flore saprophyte adjacente. Le glycolate de guanidine est de préférence présent à une concentration d'environ 0,1%, dans des compositions sous forme de stick, solution pour spray ou bille.

Par ailleurs, le glycolate de guanidine potentialise les effets d'autres actifs en dermo cosmétologie.

On pourra en particulier l'associer dans des compositions, utiles en particulier à titre de médicaments, à des corticoïdes topiques ou à l'hydroquinone.

Le glycolate de guanidine peut avantageusement être utilisé en cosmétologie.

Plus particulièrement l'invention a pour objet une méthode pour améliorer l'aspect de la peau et/ou des cheveux, caractérisée en ce qu'on applique localement une composition contenant un sel d'acide $\alpha$ hydroxylé et de guanidine tel que définie précédemment.

Les compositions contenant du glycolate de guanidine selon l'invention permettent notamment de diminuer les tiraillements et les signes associés à la sécheresse cutanée et/ou les changements de la peau liés au vieillissement. Elles permettent notamment d'améliorer l'aspect de la peau dans les cas de rides, perte d'élasticité, ainsi que les taches de vieillesse.

Les exemples qui suivent sont destinés à illuster l'invention sans aucunement en limiter la portée.

Dans ces exemples, on se réferera aux figures suivantes :

- Figure 1 : Contenu en eau des échantillons de stratum corneum traité par différentes compositions.
- Figure 2 : évaluation par spectroscopie infrarouge de l'effet hydratant de trois émulsions contenant respectivement l'excipient seul, 13% de glycolate de guanidine et 13% de lactate d'ammonium.
- Figure 3 : évaluation par spectroscopie infrarouge de l'effet hydratant de deux émulsions contenant respectivement 5% de glycérine et 5% de glycolate de guanidine.

**Exemple 1 : Préparation du glycolate de guanidine**

76 g d'acide glycolique (1 mole) et 90 de carbonate de guanidine (0,5 mole) sont ajoutés dans un litre d'eau sous forte agitation. Après évaporation sous pression réduite, on obtient quantitativement le glycolate de guanidine de formule :

$$HO - CH_2 - CO_2^{\ominus}$$

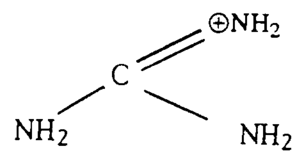

Formule brute : $C_3H_9N_3O_3$
Masse moléculaire : 135

| Analyse centésimale | | | |
|---|---|---|---|
| | C | H | N |
| % calculés | 26,66 | 6,66 | 31,11 |
| % trouvés | 26,70 | 6,7 | 31,2 |

**Spectre IR**

Bandes caractéristiques de l'ion guanidium à 3 365 et 3 171 $cm^{-1}$.

Les autres sels de guanidine avec les acides alpha ou bêta hydroxylés sont préparés selon le même mode opératoire en tenant compte de la valence de ces acides.

**Exemple 2 : Capacité de rétention sur des lambeaux de peau**

Des échantillons de stratum corneum sont immergés pendant 24 heures dans une solution de l'actif à analyser. Ils sont ensuite séchés pendant 24 heures dans un dessicateur sur silicagel puis réhydratés pendant 48 heures dans une enceinte à 90% d'humidité relative.

Ces échantillons sont analysés après l'étape de séchage et après l'étape de réhydratation. La technique utilisée est la chromatographie en phase gazeuse couplée à la spectrométrie de masse.

Les échantillons de peau pesés sont placés dans un four chauffé à 100°C, la vapeur d'eau désorbée est entrainée par un gaz de balayage sec vers le chromatographe où elle sera détectée.

Un spectre est obtenu en sortie de l'appareil. L'eau désorbée est quantifiée par la mesure de l'aire du pic. Le spectromètre de masse couplé à la CPG permet de s'assurer que le composé désorbé et quantifié est bien de l'eau.

Cette technique allie donc une analyse qualitative à une analyse quantitative. Les résultats sont donnés en pourcentage d'eau retenue et permettent donc d'évaluer le pouvoir hygroscopique des produits.

Les résultats sont présentés sur la figure 1.

Dans les conditions expérimentales, la peau traitée avec une solution aqueuse à 13% de glycolate de guanidine retient 300% d'eau, dans une ambiance de 90% d'humidité relative. Dans les mêmes conditions, la peau traitée avec une solution à 13% de lactate d'ammonium ne retient que 135% d'eau et celle traitée avec une solution à 15% de glycérol, 125% d'eau.

Les résultats sont donc largement en faveur du glycolate de guanidine.

**Exemple 3 : Hydratation in vivo mesurée par spectroscopie infrarouge**

La spectroscopie infrarouge in vivo est une technique non invasive, rapide et de grande sensibilité. Les molécules absorbent toutes différemment le rayonnement infrarouge. C'est en analysant leur spectre d'absorption que l'on peut retrouver la structure de la molécule.

En fonction de l'application de divers traitements, des liaisons entre l'eau apportée et la peau se créent et entraînent des modifications de certaines bandes d'absorption.

Ainsi seront analysés sur le spectre les pics suivants :

- le pic d'absorption dû à l'élongation de la liaison OH de l'eau à 2100 cm-1. Les variations d'intensité de ce pic ne sont pas visibles à l'oeil nu mais elles sont significatives et donc enregistrées.
- le pic d'absorption dû à l'élongation des liaisons amide I à 1640 cm-1. L'élongation de cette liaison se trouve modifiée lorsque des liaisons hydrogènes se créent par apport d'eau.
- le pic d'absorption dû à l'élongation des liaisons amide II à 1540 cm-1. Ce pic n'est influencé par la présence d'eau que de façon négligeable ; il est donc utilisé pour pondérer les deux pics précédents.

Les préparations cosmétiques à tester sont appliquées sur une zone délimitée de l'avant-bras. Une zone non traitée est conservée comme témoin.

Pour chaque spectre obtenu sont mesurés les rapports suivants :

$$\frac{\text{amideI}}{\text{amideII}}$$ appelé facteur d'hydratation (ou MF)

$$\frac{eau}{amideI}$$

Ces rapports sont calculés aux temps 0, 1, 2, ..., 6 heures et les courbes suivantes sont tracées :

MF témoin(t) - MF bras traité(t) = f(t) avec t, représentant le temps en heures.

$$\frac{eau}{amideI}\text{ témoin(t) - }\frac{eau}{amideI}\text{ bras traité(t) = f(t)}$$

L'allure de ces courbes est ensuite comparée. En effet, le pic des amides I pourrait être directement influencé par le produit appliqué alors que le pic à 2100 cm-1 n'est influencé que par l'eau.

Si les deux courbes obtenues évoluent dans le même sens, on peut considérer que la variation enregistrée rend bien compte de la fixation de l'eau sur la peau. Dans le cas contraire, il faut émettre des réserves sur les conclusions à apporter.

On teste par cette technique des crèmes contenant 5% et 13% de glycolate de guanidine, de formules suivantes (les pourcentages sont exprimés en p/p) :

| A | |
|---|---|
| 1. Glycolate de guanidine | 13% |
| 2. Glycérol | 15% |
| 3. Paraffine liquide | 8% |
| 4. Cétéaryl glucoside | 5% |
| 5. Huile minérale/alcools de lanoline | 2% |
| 6. Cyclométhicone | 2,5% |
| 7. Polyacrylamide | 1% |
| 8. Parfum | 0,075% |
| 9. $H_2O$ qsp | 100 |
| | pH = 5,5 |

| B | |
|---|---|
| 1. Glycolate de guanidine | 5% |
| 2. Glycérol | 15% |
| 3. Paraffine liquide | 8% |
| 4. Cétéaryl glucoside | 5% |
| 5. Huile minérale/alcools de lanoline | 2% |
| 6. Cyclométhicone | 2,5% |
| 7. Polyacrylamide | 1% |
| 8. Parfum | 0,075% |
| 9. $H_2O$ qsp | 100 |

(suite)

| | |
|---|---|
| B | |
| | pH = 5,5 |

Les effets sont comparés avec ceux de compositions contenant :

- 15% glycérol
- 5% glycérol
- 13% lactate d'ammonium
- 5% lactate d'ammonium.

Les résultats sont présentés sur la figure 2.

Dans ces conditions expérimentales, la crème contenant 13% de glycolate de guanidine est beaucoup plus hydratante que le placebo et que la crème contenant 13% de lactate d'ammonium qui constitue une référence. La différence d'hydratation est remarquable et se maintient dans le temps au delà de 6 heures.

La figure 3 montre que la crème contenant 5% de glycolate de guanidine est beaucoup plus hydratante que celle contenant 5% de glycérol qui est une référence dans le domaine de l'hydratation. De la même façon que précédemment, la différence d'hydratation est remarquable et se maintient dans le temps au delà de 6 heures.

**Exemple 4 : Formule 1**

| | |
|---|---|
| 1. Glycolate de Guanidine | 1 à 14% |
| 2. Glycérol | 15% |
| 3. Huile de paraffine | 8% |
| 4. Cétéaryl glucoside | 5% |
| 5. Huile minérale/Alcools de lanoline | 2% |
| 6. Cyclométhicone | 2,5% |
| 7. Polyacrylamide | 1% |
| 8. Parfum | 0,075% |
| 9. $H_2O$ qsp | 100 |
| | pH = 5,5 |

**Exemple 5 : Formule 2**

| | |
|---|---|
| 1. Lactate de Guanidine | 1 à 14% |
| 2. Glycérol | 15% |
| 3. Vaseline | 8% |
| 4. Huile minérale/Alcools de lanoline | 2% |
| 5. Cyclométhicone | 2,5% |
| 6. Stéarate de glycérol/Lauryl sulfate de sodium | 10% |
| 7. Polyacrylamide | 3% |
| 8. Parfum | 0,1% |
| 9. $H_2O$ qsp | 100 |

(suite)

| | |
|---|---|
| | pH = 5,3 |

**Exemple 6 : Formule 3**

| | |
|---|---|
| 1. Glycolate de Guanidine | 1 à 14% |
| 2. Glycérol | 15% |
| 3. Vaseline | 8% |
| 4. Monostéarate de glycérol et POEG | 5% |
| 5. Huile minérale/Alcools de lanoline | 2% |
| 6. Cyclométhicone | 2,5% |
| 7. Stéarate de glycérol/Lauryl sulfate de sodium | 10% |
| 8. Alcool cétylique | 0,5% |
| 9. $H_2O$ qsp | 100 |
| | pH = 5,5 |

**Exemple 7 : Formule 4**

| | |
|---|---|
| 1. Glycolate de Guanidine | 1 à 14% |
| 2. Glycérol | 15% |
| 3. Vaseline | 8% |
| 4. Monostéarate de glycérol et POEG | 5% |
| 5. Polyacrylamide | 2% |
| 6. C 10-30 cholestérol/Lanostérol ester | 4% |
| 7. Volatile silicone et Polydiméthylsiloxane | 3% |
| 8. $H_2O$ qsp | 100 |
| | pH = 5,2 |

**Exemple 8 : Formule 5**

| | |
|---|---|
| 1. Lactate de Guanidine | 1 à 14% |
| 2. Glycérol | 15% |
| 3. Vaseline | 8% |
| 4. Monostéarate de glycérol et POEG | 5% |
| 5. Acide stéarique | 7% |
| 6. Alcool cétostéarylique | 0,5% |

(suite)

| | |
|---|---|
| 7. $H_2O$ qsp | 100<br>pH = 5 |

**Exemple 9 : Formule 6**

| | |
|---|---|
| 1. Glycolate de Guanidine | 1 à 14% |
| 2. Méthylglucose sesquistéarate | 2% |
| 3. Méthylglucose sesquistéarate éthoxylé (20 O-E) | 2% |
| 4. Glycérol | 15% |
| 5. Vaseline | 8% |
| 6. Squalane | 11% |
| 7. Polyacrylamide | 5% |
| 8. $H_2O$ qsp | 100<br>pH = 4 |

## Revendications

**1.** Composition dermato-cosmétologique, caractérisée en ce qu'elle contient à titre de principe actif au moins un sel susceptible d'être obtenu par réaction de la guanidine ou d'un de ses sels et de l'acide glycolique.

**2.** Composition selon la revendication 1, caractérisée en ce que la concentration en acide glycolique est comprise entre 5 et 70% en poids par rapport au poids total de la composition.

**3.** Composition selon la revendication 1, caractérisée en ce qu'elle contient à titre de principe actif du glycolate de guanidine.

**4.** Composition selon la revendication 3, caractérisée en ce que le glycolate de guanidine est présent à une concentration comprise entre 0,5% et 15% en poids par rapport au poids total de la composition.

**5.** Composition selon l'une des revendications 1 à 4, caractérisée en ce qu'elle a un pH compris entre 3,5 et 6,5.

**6.** Composition selon l'une des revendications 1 à 5, caractérisée en ce qu'elle contient du glycolate de guanidine à titre de conservateur.

**7.** Utilisation d'une composition selon l'une des revendications 1 à 6, pour la préparation d'un médicament destiné au traitement d'une affection choisie dans le groupe comprenant : acné, ichtyose, kératoses séborrhéiques, kératoses actiniques, lentigos séniles, verrues et hyperkératinisations.

**8.** Utilisation selon la revendication 9, caractérisée en ce que la composition contient en outre au moins un autre principe actif en particulier un corticoïde et/ou de l'hydroquinone.

**9.** Méthode non-thérapeutique pour améliorer l'aspect de la peau et/ou des cheveux, caractérisée en ce qu'on applique localement une composition selon l'une des revendications 1 à 6.

**10.** Méthode non-thérapeutique selon la revendication 9, caractérisée en ce qu'on diminue les troubles des peaux sèches et/ou les changements de la peau liés au vieillissement.

## Claims

1. Dermocosmetic composition, characterized in that it contains, as active principle, at least one salt capable of being obtained by reacting guanidine or one of its salts with glycolic acid.

2. Composition according to Claim 1, characterized in that the glycolic acid concentration is between 5 and 70% by weight relative to the total weight of the composition.

3. Composition according to Claim 1, characterized in that it contains, as active principle, guanidine glycolate.

4. Composition according to Claim 3, characterized in that guanidine glycolate is present at a concentration of between 0.5% and 15% by weight relative to the total weight of the composition.

5. Composition according to one of Claims 1 to 4, characterized in that it has a pH of between 3.5 and 6.5.

6. Composition according to one of Claims 1 to 5, characterized in that it contains guanidine glycolate as preservative.

7. Use of a composition according to one of Claims 1 to 6, for the preparation of a medicament intended for the treatment of a condition chosen from the group comprising: acne, ichthyosis, seborrhoeic keratoses, actinic keratoses, senile lentigines, verrucas and hyperkeratinizations.

8. Use according to Claim 9, characterized in that the composition contains, in addition, at least one other active principle, in particular a corticoid and/or hydroquinone.

9. Non-therapeutic method for improving the appearance of the skin and/or the hair, characterized in that a composition according to one of Claims 1 to 6 is applied locally.

10. Non-therapeutic method according to Claim 9, characterized in that dry skin disorders and/or skin changes related to ageing are reduced.

## Patentansprüche

1. Dermato-kosmetologische Zusammensetzung, dadurch gekennzeichnet, daß sie als wirksames Prinzip mindestens ein durch Reaktion von Guanidin oder einem seiner Salze und Glykolsäure erhältliches Salz enthält.

2. Zusammensetzung nach Anspruch 1, dadurch gekennzeichnet, daß die Konzentration an Glykolsäure von 5 bis 70 Gew.-%, bezogen auf das Gesamtgewicht der Zusammensetzung, beträgt.

3. Zusammensetzung nach Anspruch 1, dadurch gekennzeichnet, daß sie als wirksames Prinzip Guanidinglykolat enthält.

4. Zusammensetzung nach Anspruch 3, dadurch gekennzeichnet, daß das Guanadinglykolat in einer Konzentration von 0,5 bis 15 Gew.-%, bezogen auf das Gesamtgewicht der Zusammensetzung, vorliegt.

5. Zusammensetzung nach einem der Ansprüche 1 bis 4, dadurch gekennzeichnet, daß sie einen pH-Wert von 3,5 bis 6,5 aufweist.

6. Zusammensetzung nach einem der Ansprüche 1 bis 5, dadurch gekennzeichnet, daß sie Guanidinglykolat als Konservierungsmittel enthält.

7. Verwendung einer Zusammensetzung nach einem der Ansprüche 1 bis 6 zur Herstellung eines Arzneimittels, bestimmt zur Behandlung einer Erkrankung ausgewählt aus der Gruppe von: Akne, Ichthyosis, seborrhoischen Keratosen, aktinischen Keratosen, Altersleberflecken, Warzen und Hyperkeratinisationen.

8. Verwendung nach Anspruch 9, dadurch gekennzeichnet, daß die Zusammensetzung außerdem mindestens ein weiteres wirksames Prinzip, insbesondere ein Corticoid und/oder Hydrochinon, enthält.

9. Nicht-therapeutisches Verfahren zur Verbesserung des Aussehens der Haut und/oder des Haares, dadurch

gekennzeichnet, daß man eine Zusammensetzung gemäß einem der Ansprüche 1 bis 6 lokal aufträgt.

10. Nicht-therapeutisches Verfahren nach Anspruch 9, dadurch gekennzeichnet, daß man Störungen trockener Haut und/oder Veränderungen der Haut, die altersbedingt sind, verringert.

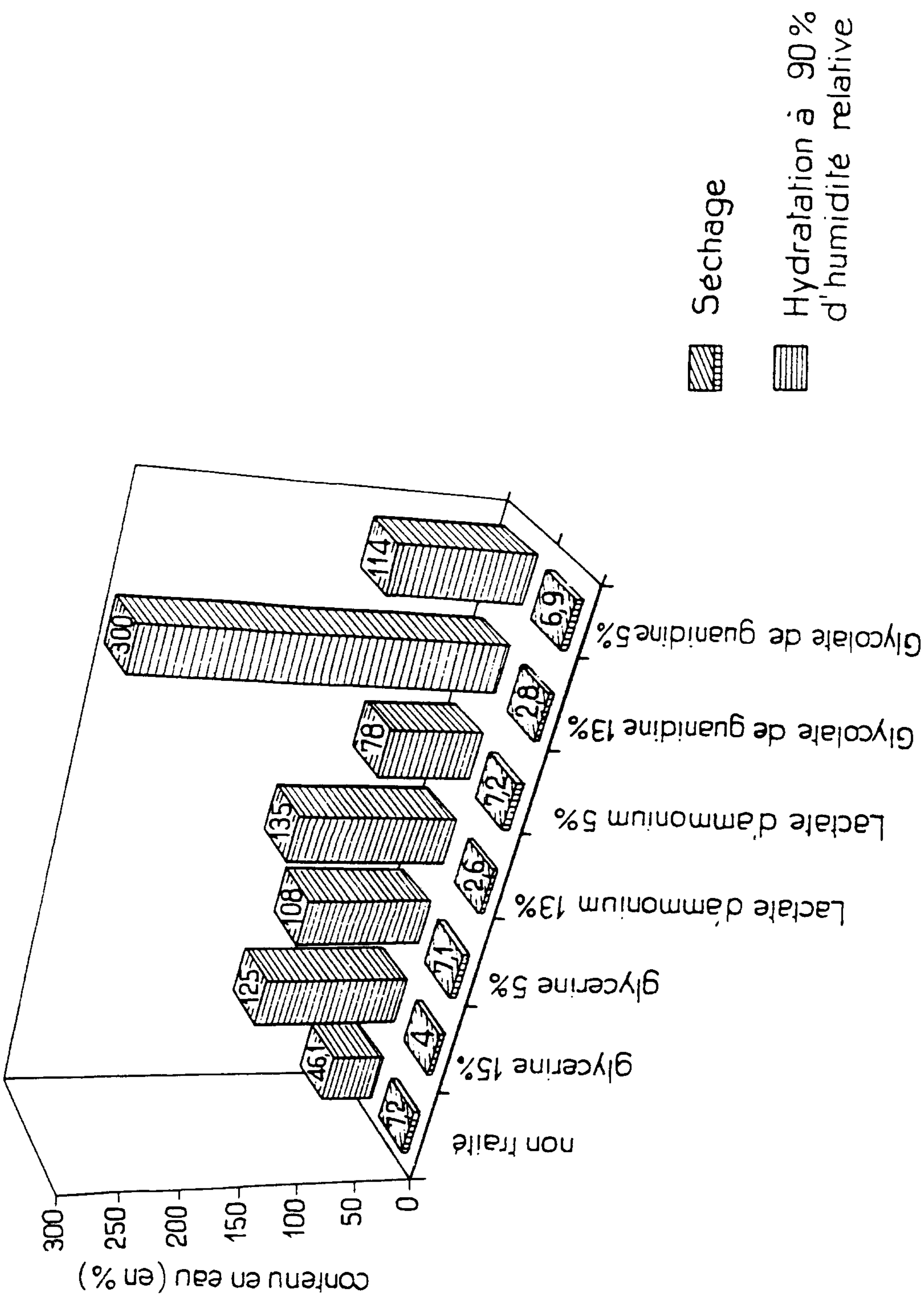
contenu en eau (en %)
300
250
200
150
100
50
0
non traité
glycerine 15%
glycerine 5%
Lactate d'ammonium 13%
Lactate d'ammonium 5%
Glycolate de guanidine 13%
Glycolate de guanidine 5%
7,2
46,1
4
125
7,1
108
2,6
135
7,2
78
2,8
300
6,9
114
Séchage
Hydratation à 90 % d'humidité relative

FIG_1

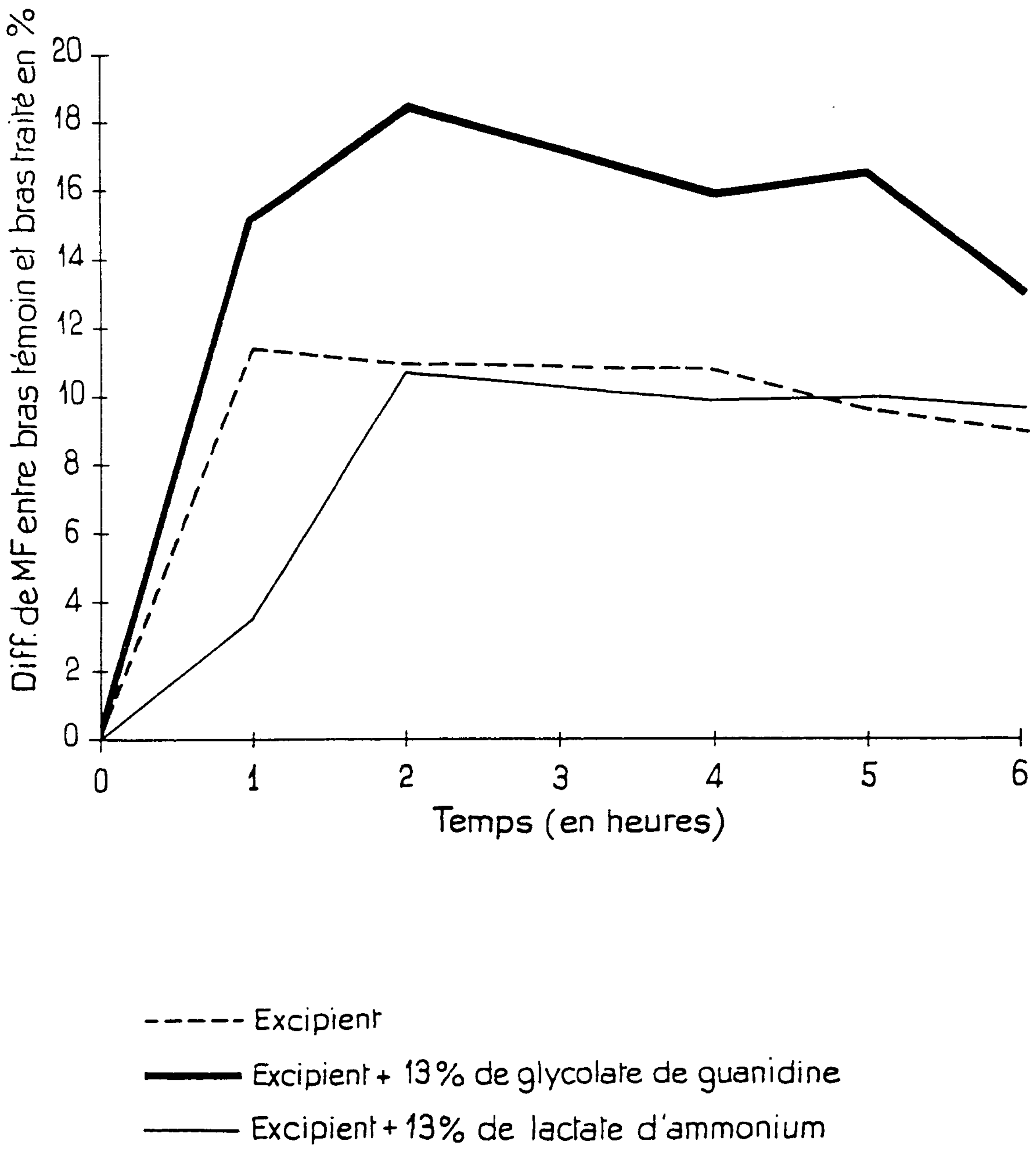
Diff. de MF entre bras témoin et bras traité en %
20
18
16
14
12
10
8
6
4
2
0
0
1
2
3
4
5
6
Temps (en heures)
----- Excipient
Excipient + 13% de glycolate de guanidine
Excipient + 13% de lactate d'ammonium

FIG_2

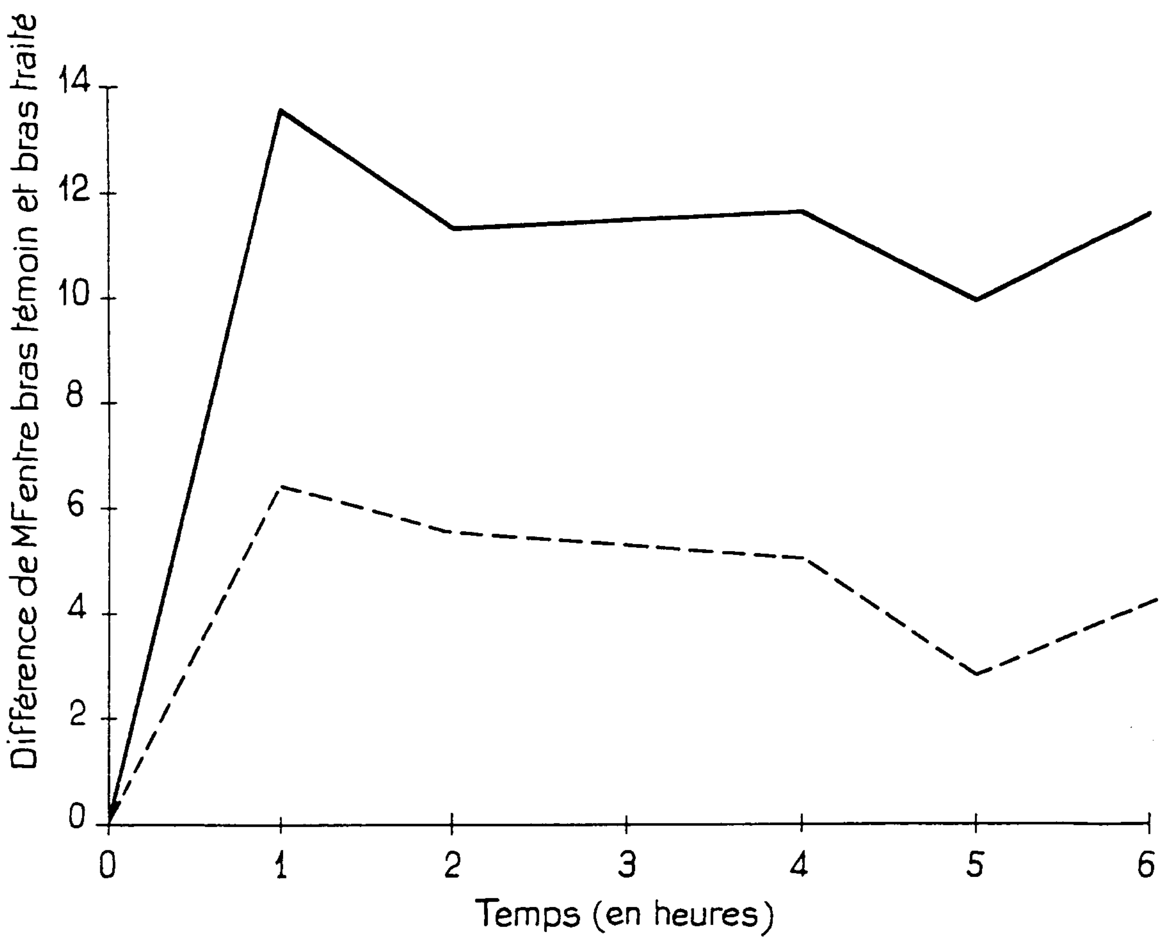


----- Excipient + 5% glycérine

——— Excipient + 5% glycolate de guanidine

FIG_3